# FASCICULE DE BREVET EUROPEEN

(11) **EP 0 939 670 B1**
(45) Date de publication et mention de la délivrance du brevet: **16.08.2001**
(21) Numéro de dépôt: 97947101.8
(22) Date de dépôt: 20.11.1997
(51) Int. Cl.: B01F 17/00

(54) **NOUVELLES COMPOSITIONS A BASE D'ALKYLGLYCOSIDES ET D'ALCOOLS GRAS**
NEUARTIGE ZUSAMMENSETZUNGEN AUF BASIS VON ALKYLGLYKOSIDEN UND FETTALKOHOLEN
NOVEL COMPOSITIONS WITH ALKYLGLYCOSIDES AND FATTY ALCOHOLS BASE

(30) Priorité: 22.11.1996 FR 9614283
(43) Date de publication de la demande: 08.09.1999
(73) Titulaire: SOCIETE D'EXPLOITATION DE PRODUITS POUR LES INDUSTRIES CHIMIQUES, S.E.P.P.I.C., 75321 Paris Cédex 07 (FR)
(72) Inventeur: MILIUS, Alain, F-06000 Nice (FR); MICHEL, Nelly, F-94700 Maisons Alfort (FR); AMALRIC, Chantal, F-81700 Blan (FR)
(74) Mandataire: Hubert, Philippe
(86) Numéro de dépôt international: FR9702091
(87) Numéro de publication internationale: WO9822207

(56) Documents cités:
- WO-A-92/06778
- WO-A-95/13863

## Description

La présente invention a pour objet de nouvelles compositions à base d'alkylglycosides et d'alcools gras, notamment utiles pour la préparation d'émulsions fluides parfaitement stables dans le temps, et l'utilisation de ces émulsions pour la préparation de compositions cosmétiques.

Les alkylglycosides ou alkylpolyglycosides (APG) sont des tensio-actifs non-ioniques qui peuvent être utilisés seuls, ou en association avec d'autres tensio-actifs, dans une large gamme d'applications industrielles et notamment en détergence ou en cosmétique.

La demande de brevet WO 92/06778, au nom de la demanderesse, décrit des mélanges d'alkylglycosides et d'alcools gras utiles en tant qu'agents auto-émulsionnables.

Plus précisément, ces mélanges comprennent :
- de 60 à 90% en poids d'au moins un alcool gras ayant de 12 à 22 atomes de carbone ; et
- de 10 à 40% en poids d'un alkylglycoside, dont la partie alkyle est de préférence identique à celle de l'alcool gras.

Ce document décrit à l'exemple 2 l'utilisation d'une composition auto-émulsionnable essentiellement constituée d'alcool oléique et d'oléylglycoside.

Par ailleurs, la demande de brevet WO 95/13863, au nom de la demanderesse, décrit des compositions à base d'alkylglycosides et d'alcools gras se présentant sous forme de concentrés, notamment utiles pour la préparation d'émulsions fluides.

Ces compositions sont essentiellement caractérisées par le fait qu'elles comprennent un mélange d'au moins deux alkylglycosides se différenciant par la nature de leur partie alkyle.

Il est précisé que l'un de ces alkylglycosides comporte une chaîne alkyle ayant 16 à 22 atomes de carbone, et de préférence 16 à 18 atomes de carbone.

Dans tous les exemples, l'alkylglycoside ayant 16 atomes de carbone est l'hexadécylglycoside, et l'alkylglycoside ayant 18 atomes de carbone est l'octadécylglycoside.

La présente invention a pour but de résoudre le problème technique consistant en la fourniture de nouvelles compositions permettant d'obtenir des émulsions dont les stabilités sont significativement améliorées par rapport à celles des compositions décrites dans l'état de la technique dont le contenu a été rappelé précédemment.

La solution, conforme à la présente invention, pour résoudre ce problème technique consiste en de nouvelles compositions, notamment utiles pour la préparation d'émulsions fluides comprenant :
i) de 30 à 90% en poids d'un mélange d'au moins un alkylglycoside de formule (I) et d'au moins un alkylglycoside de formule (II) :

   RO(G)ₓ (I)

   R'O(G')_{x'} (II)

   dans lesquelles :
   - R représente un radical aliplhatique, linéaire ou ramifié, saturé ou insaturé, ayant de 8 à 22 atomes de carbone ;
   - R' représente un radical oléyle ou isostéaryle ;
   - G et G', identiques ou différents, représentent un reste de saccharide ; et
   - x et x', identiques ou différents, représentent un nombre compris entre 1 et 10 ; étant précisé que le (ou les) alkylglycoside(s) de formule (II) représente(nt) 25 à 60% en poids dudit mélange ;
ii) de 10 à 70% en poids d'au moins un alcool gras de formule R₁OH dans laquelle R₁ représente un radical aliphatique, linéaire ou ramifié, saturé ou insaturé, ayant de 8 à 22 atomes de carbone.

Il a donc été découvert, de façon tout à fait surprenante et inattendue, que des compositions comportant un alcool gras et un mélange d'alkylglycosides comprenant de 25 à 60% en poids d'oléylglycoside et/ou d'isostéarylglycoside permettent d'obtenir des émulsions fluides, remarquablement stables pendant au moins trois mois.

Il a également été découvert, de façon tout à fait inattendue, que contrairement aux compositions à base d'alkylglycoside et d'alcools gras connues par le document WO 92/06778, les compositions conformes à la présente invention permettent d'obtenir des émulsions fluides en présence de phases grasses et notamment d'isononanoate d'isononyle ou d'huile de paraffine.

Les alkylglycosides de formules (I) et (II) précitées peuvent comporter, à titre de restes de saccharides représentés par les lettres G et G', un reste de glucose ou dextrose, saccharose, fructose, galactose, maltose, maltotriose, lactose, cellobiose, mannose, ribose, dextrane, talose, allose, xylose, levoglucosane, cellulose et amidon.

Avantageusement, G et G' représenteront chacun un reste glucose.

Il est en outre à noter que chaque unité de la partie polyoside de l'alkylglycoside peut être sous forme anomérique α ou β, sous forme L ou D, et la configuration du reste de saccharide peut être de type furanoside ou pyranoside.

Les indices x et x' représentent le degré de polymérisation moyen du reste de saccharide. Ces indices représentent un nombre compris entre 1 et 10, de préférence entre 1,05 et 2,5.

L'expression "alkylglycoside" utilisée dans le cadre de la présente invention désigne donc aussi bien les alkylmonoosides (x ou x' = 1) que des alkylpolyosides (x ou x'>1).

Les alkylglycosides de formule (II) représenteront de préférence 25 à 60% en poids et encore de préférence 35 à 50% en poids du mélange d'alkylglycosides présent au sein de la composition.

Selon un second aspect, la présente demande vise à couvrir des émulsions comprenant au moins une phase aqueuse et une phase huileuse, ladite émulsion comprenant une composition émulsionnante telle que décrite ci-dessus.

D'une façon générale, une telle émulsion comprendra de 1 à 25% en poids, et de préférence de 1 à 10% en poids de la composition émulsionnante précitée.

Eventuellement, cette émulsion peut également comprendre un agent émulsionnant complémentaire en une quantité telle que la quantité totale d'agents émulsionnants au sein de l'émulsion soit inférieure ou égale à 25 % en poids.

Un tel agent émulsionnant complémentaire peut être par exemple ester de sorbitan, polysorbate, ester de sucrose, ester de polyglycérol, ester phosphorique, alkylsulfate, acide gras éthoxylé, alcool gras éthoxylé.

La phase huileuse constitutive de l'émulsion peut être constituée par le ou les alcools gras constitutifs de la composition émulsionnante de l'invention, sans qu'il ne soit nécessaire de mettre en oeuvre une autre huile. Mais plus généralement, on utilisera une huile choisie parmi les huiles suivantes :
- les huiles d'origine végétale, telles que l'huile d'amandes douces, l'huile de coprah, l'huile de ricin, l'huile de jojoba, l'huile d'olive, l'huile de colza, l'huile d'arachide, l'huile de tournesol, l'huile de germes de blé, l'huile de germes de maïs, l'huile de soja, l'huile de coton, l'huile de luzerne, l'huile de pavot, l'huile de potimarron, l'huile d'onagre, l'huile de millet, l'huile d'orge, l'huile de seigle, l'huile de carthame, l'huile de bancoulier, l'huile de passiflore, l'huile de noisette, l'huile de palme, le beurre de karité, l'huile de noyau d'abricot, l'huile de calophyllum ;
- les huiles d'origine animale, telles que le perhydrosqualène, le squalène ;
- les huiles minérales, telles que l'huile de paraffine ou huile de vaseline, et les huiles minérales, notamment issues 'de coupes pétrolières, telles que les isoparaffines, ayant un point d'ébullition compris entre 300 et 400°C ;
- les huiles synthétiques, notamment les esters d'acides gras tels que le myristate de butyle, le myristate de propyle, le myristate de cétyle, le palmitate d'isopropyle, le stéarate de butyle, le stéarate d'hexadécyle, le stéarate d'isopropyle, le stéarate d'octyle, le stéarate d'isocétyle, l'oléate dodécyle, le laurate d'hexyle, le dicaprylate de propylèneglycol, les esters dérivés d'acide lanolique, tels que le lanolate d'isopropyle, le lanolate d'isocétyle, les triglycérides comme le triheptanoate de glycérol, lies alkylbenzoates, les isoparaffines, les poly-alphaoléfines, les polyoléfines, les isoalcanes de synthèse comme l'isohexadecane, l'isododécane et les huiles de silicone. Parmi ces dernières, on peut plus particulièrement citer les diméthylpolysiloxanes, méthylphénylpolysiloxanes, les silicones modifiés par des amines, les silicones modifiés par des acides gras, les silicones modifiés par des alcools, les silicones modifiés par des alcools et des acides gras, des silicones modifiés par des groupements polyéther, des silicones époxy modifiés, des silicones modifiés par des groupements fluorés, des silicones cycliques et des silicones modifiés par des groupements alkyles.

D'une façon générale, les émulsions conformes à la présente invention comprendront jusqu'à 50% en poids de phase huileuse telle que définie précédemment.

Ces émulsions peuvent être préparées par simple dispersion d'une phase grasse constituée de la composition émulsionnante décrite et éventuellement d'une ou plusieurs huiles telles que décrites ci-dessus, dans une phase hydrophile, généralement de l'eau ou un solvant hydrophile.

La dispersion peut être réalisée à chaud ou à froid en fonction du point de fusion de la composition émulsionnante, tous les constituants devant être liquides au moment du mélange.

L'invention sera illustrée plus en détail par les exemples suivants, donnés uniquement à titre illustratif.

Dans ces exemples, les pourcentages sont exprimés en poids et la température est la température ambiante, sauf indication contraire.

### EXEMPLE 1

### Procédé de préparation d'une composition selon l'invention

On introduit dans un réacteur polyvalent une coupe d'alcools gras ayant la composition suivante (pourcentage en poids) :
C10:9%,
C12 : 12 %,
C14 : 18,5 %,
C16 : 6%,
C18 : 5,3 %,
alcool oléique : 49,2 %.

On introduit également dans le réacteur du glucose, de sorte que le rapport molaire entre les alcools gras et le glucose soit de : 6/1.

On fait ensuite réagir le glucose avec les alcools gras pendant 5 heures à une température comprise entre 100 et 105°C, en présence d'acide sulfurique en tant que catalyseur.

La réaction est réalisée sous un vide partiel de 15mm de mercure.

Après réaction, on neutralise le catalyseur au moyen d'une base.

Les alcools présents dans le mélange réactionnel, sont partiellement éliminés par distillation sur un évaporateur à film mince.

La composition obtenue comprend :
58,7 % d'alcools gras libres :
C12 : 0,5 %,
C14 : 3,4 %,
C16 : 1,4 %,
C18 : 5,3 %,
alcool oléique : 48,1 %
et
41,3 % d'alkylglycosides présentant la répartition suivante (% en poids) :
C10 glucosides : 11,1 %,
C12 glucosides : 11,8 %,
C14 glucosides : 20,6 %,
C 16 glucosides : 3,8 %,
C 18 glucosides : 5,2 %,
oléylglucosides : 47,5 %.

### EXEMPLES 2 à 5

### Procédé de préparation de compositions selon l'invention.

En suivant le protocole expérimental décrit à l'exemple 1, on a préparé les quatre compositions suivantes, conformes à la présente invention :

### Composition selon l'exemple 2

Alcools gras de départ : identiques à ceux de l'exemple 1

La composition obtenue comprend :
17,4 % d'alcools gras libres :
C12 : 0,4 %,
C14 : 1,3 %,
C16 : 0,4 %,
C18 : 0,2 %,
alcool oléique : 15,1 %
et
82,6 % d'alkylglucosides ayant une répartition identique à celle décrite dans l'exemple 1.

### Composition selon l'exemple 3

Les alcools gras de départ ont la composition suivante :
C12 : 6,5 %,
C14 : 17,5 %,
C16 : 13,5 %,
C18 : 17,5 %,
alcool oléique : 45 %.

La composition obtenue comprend :
29,2 % d'alcools gras libres :
C12 : 0,4 %,
C14: 1,7%,
C16 : 2,8 %,
C18 : 7,8 %,
alcool oléique : 16,5 %
et
70,8 % d'alkylglucosides ayant la répartition suivante :
C12 glucosides : 8,9 %,
C14 glucosides : 18,1 %,
C16 glucosides : 16 %,
C18 glucosides : 16 %,
oléylglucosides : 41 %.

### Composition selon l'exemple 4

On ajoute à la composition obtenue selon l'exemple 3 des alcools gras C16, C18 et oléique.

La composition ainsi obtenue comprend :
39 % d'alcools gras :
C12 : 0,3 %,
C14 : 1,4 %,
C16 : 4,9 %,
C18 : 9,7 %,
alcool oléique : 22,7 %
et
61 % d'alkylglycosides ayant la même répartition que celle indiquée dans l'exemple 3.

### Composition selon l'exemple 5

On ajoute à la composition obtenue selon l'exemple 3 des alcools gras C18 et oléique.

La composition ainsi obtenue comprend :
51,2 % d'alcools gras :
C12 : 0,2 %,
C14 : 1,1 %,
C16 : 3,9 %,
C18 : 27,8 %,
alcool oléique : 18,2 %
et
48,8 % d'alkylglycosides dont la répartition est identique à celle de l'exemple 3.

### EXEMPLE 6

### Procédé de préparation d'émulsions fluides à partir d'une composition selon l'invention

On prépare diverses émulsions fluides au moyen de la composition émulsionnante obtenue à l'exemple 1, lesdites émulsions présentant les compositions suivantes :

| | |
|---|---|
| Composition émulsionnante | 5 % |
| Huile ou mélange d'huiles | 0/10/30/50 % |
| Eau | QSP 100 % |

Ces émulsions ont été préparées avec un appareil Silverson L 4 R. Les deux phases (huileuse et aqueuse) sont préalablement chauffées à 70°C, puis homogénéisées 4 minutes à 4.000 rpm. Les émulsions sont ensuite refroidies jusqu'à température ambiante sous agitation lente type ancre.

Deux huiles ont été utilisées :
. PRIMOL® 352 - Huile de paraffine.
. LANOL® 99 - Isononanoate d'isononyle.

### EXEMPLE 7

### Mise en évidence des propriétés de stabilité des émulsions fluides obtenues par la mise en oeuvre des compositions selon l'invention, par comparaison aux compositions de l'état de la technique.

En suivant le protocole expérimental décrit à l'exemple 6, on a préparé différentes émulsions à partir des compositions selon l'invention décrites aux exemples 1 à 5.

### Exemples de comparaison

Pour mettre en évidence les propriétés remarquables de stabilité des émulsions fluides préparées par la mise en oeuvre des compositions selon l'invention, on a préparé différentes émulsions de comparaison en utilisant le protocole décrit à l'exemple 6, au moyen de compositions de l'état de la technique respectivement désignées A, B, C, D et de deux compositions de comparaison respectivement désignées E et F.

Les compositions de comparaison A et B correspondent aux compositions décrites respectivement aux exemples 1 et 4 du document WO 92/06778.

La composition de comparaison C correspond à la composition décrite à l'exemple 2 du document WO 92/06778, composition dans laquelle on ne trouve qu'un seul type d'alkylglycoside à savoir l'oléylglycoside.

La composition de comparaison D correspond à la composition de l'exemple 1 du document WO 95/13863.

La composition de comparaison E est obtenue en ajoutant de l'alcool en C 18 dans la composition D.

La composition de comparaison F est obtenue en suivant le protocole opératoire décrit à l'exemple 1, sans distillation partielle des alcools.

Les alcools gras de départ ont la même composition que ceux utilisés dans l'exemple 3.

La composition obtenue comprend :
73,2 % d'alcools gras :
C12 : 8,0 %,
C14 : 15,3 %,
C16 : 10.1 %,
C18 : 12,4 %,
alcool oléique : 27,4 %
et
26,8 % d'alkylglycosides ayant la même répartition que celle décrite dans l'exemple 3.

La stabilité des émulsions obtenues au moyen des compositions selon l'invention ainsi qu'au moyen des compositions de comparaison précitées a été évaluée de façon visuelle à 23±2°C.

Les résultats obtenus ont été regroupés dans le tableau I.

Dans ce tableau, on a utilisé les abréviations suivantes :
- "DPH JX": (où X représente un nombre entier) signifie déphasage au bout de X jours.
- ">MX": (où X représente un nombre entier) signifie que l'émulsion est restée stable pendant au moins X mois.

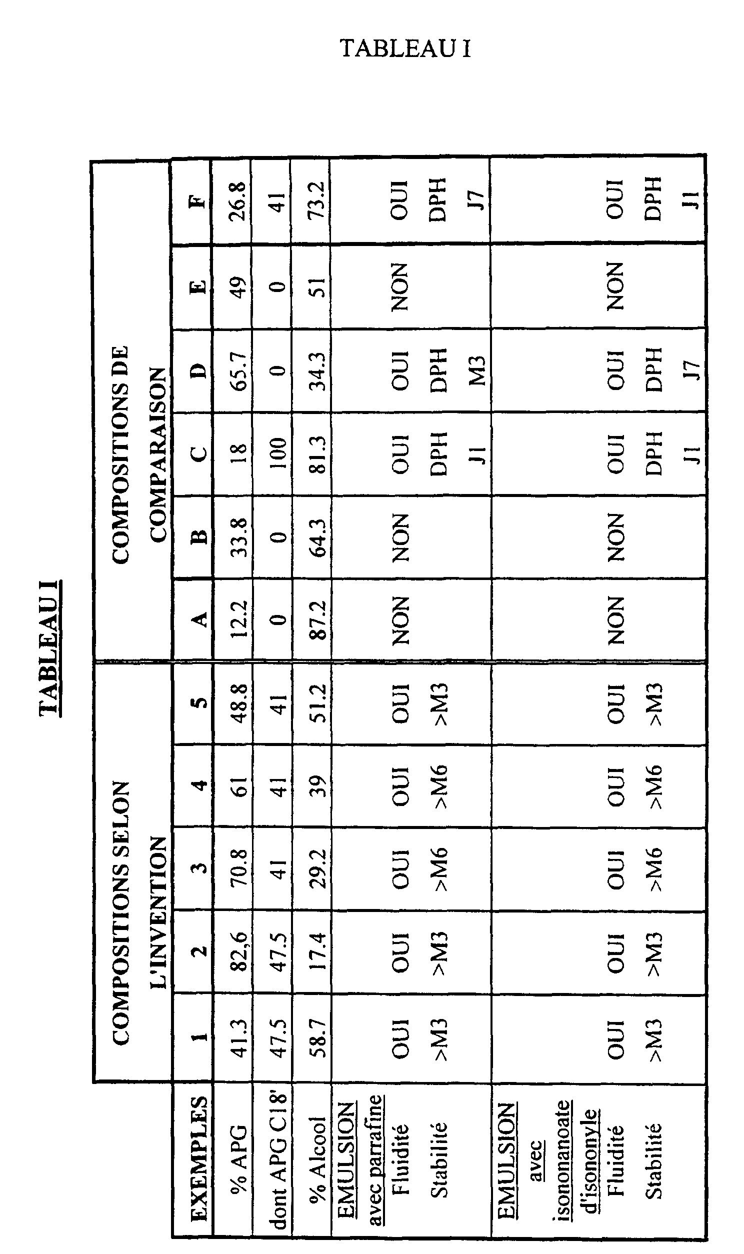

Les résultats obtenus montrent que les compositions de comparaison A et B (exemples 1 et 4 du document WO 92/06778) ainsi que la composition E ne permettent pas d'obtenir des émulsions fluides dans les conditions opératoires utilisées.

En effet, les émulsions obtenues à l'aide des ces compositions de comparaison sont épaisses dès lors que l'émulsion est réalisée en présence d'une phase grasse.

La composition de comparaison C (exemple 2 du document WO 92/06778), la composition de comparaison D (exemple 1 du document WO 95/13863) et la composition de comparaison F conduisent à des émulsions fluides, mais ces émulsions déphasent plus ou moins rapidement dans le temps.

Seules les compositions de l'invention permettent donc d'obtenir des émulsions fluides stables pendant plus de 3 mois, les compositions des exemples 3 et 4 étant même stables pendant plus de 6 mois.

Ceci démontre clairement la supériorité des compositions conformes à la présente invention vis-à-vis des compositions de l'état de la technique.

### EXEMPLE 8

### Mise en évidence des propriétés de stabilité des émulsions fluides obtenues par la mise en oeuvre des compositions selon l'invention, avec ou sans huile.

On a préparé différentes émulsions à partir de la composition selon l'invention décrite à l'exemple 3, en faisant varier la concentration en composition émulsionnante, lesdites émulsions comprenant 10% en poids d'isonanonate d'isononyle.

La stabilité des compositions ainsi réalisées a été évaluée et les résultats obtenus sont reportés au Tableau II ci-après.

D'autres exemples, dont les résultats ne sont pas rapportés ici, ont montré qu'il était possible d'obtenir des émulsions fluides et stables contenant jusqu'à 25% en poids de la composition de l'exemple 3.

Ces résultats montrent que des émulsions fluides stables peuvent être obtenues avec des compositions selon l'invention pour une large plage de concentrations variant généralement de 1 à 25%, et de préférence de 1 à 10% en poids par rapport au poids de l'émulsion.

**TABLEAU II**

| **COMPOSITION DE L'EXEMPLE 3** | **CONCENTRATION EN COMPOSITION EMULSIONNANTE** | | |
|---|---|---|---|
| | 3% | 5% | 10% |
| **STABILITE** | >M6 | >M6 | >M6 |

On a également préparé différentes émulsions à partir de la composition selon l'invention décrite à l'exemple 4, utilisée en une quantité de 5 % en poids, en faisant varier la nature de l'huile utilisée, celle-ci étant présente en une quantité de 10 % en poids.

Les résultats obtenus sont reportés au tableau III ci-après :

**TABLEAU III**

| **COMPOSITION DE L'EXEMPLE 3 (5 %)** | **TYPE D'HUILE (10 %)** | | | |
|---|---|---|---|---|
| | **Diméthyl polysiloxanne** | **Huile d'amande douce** | **Huile de paraffine** | **Triheptanoate de glycérol** |
| **STABILITE** | >M6 | >M6 | >M6 | >M6 |

Les résultats obtenus ont montré qu'il était possible d'obtenir des émulsions fluides et stables pour une large variété d'huiles.

D'autres études, dont les résultats n'ont pas été reportés ont montré qu'il était possible d'obtenir des émulsions fluides et stables à partir de compositions émulsionnantes identiques à celles décrites aux exemples 1 à 5, si ce n'est que la fraction oléylglycoside a été remplacée par une fraction isostéarylglucoside.

Ces compositions peuvent être obtenues à partir d'alcool isostéarylique encore dénommé isooctadécylique ou isooctadécanol.

De tels alcools sont, par exemple, commercialisés par la société HOECHST sous la dénomination Isooctadécanol ou par la société UNICHEMA sous la dénomination Prisorine® 3515.

Les compositions qui viennent d'être décrites trouvent application pour la préparation d'émulsions fluides dans le domaine cosmétique et en particulier les laits, notamment du type huile dans eau, à usage cosmétique ou hygiénique comme les laits démaquillants, les laits corporels ou les laits solaires.

## Revendications

1. Compositions, notamment utiles pour la préparation d'émulsions fluides comprenant :
i) de 30 à 90% en poids d'un mélange d'au moins un alkylglycoside de formule (I) et d'au moins un alkylglycoside de formule (II) :
RO(G)ₓ (I)
R'O(G')_{x'} (II)
dans lesquelles :
- R représente un radical aliphatique, linéaire ou ramifié, saturé ou insaturé, ayant de 8 à 22 atomes de carbone ;
- R' représente un radical oléyle ou isostéaryle ;
- G et G', identiques ou différents, représentent un reste de saccharide ; et
- x et x', identiques ou différents, représentent un nombre compris entre 1 et 10 ; étant précisé que le (ou les) alkylglycoside(s) de formule (II) représente(nt) 25 à 60% en poids dudit mélange ;
ii) de 10 à 70% en poids d'au moins un alcool gras de formule R₁OH dans laquelle R₁ représente un radical aliphatique, linéaire ou ramifié, saturé ou insaturé, ayant de 8 à 22 atomes de carbone.

2. Compositions selon la revendication 1, caractérisées en ce que le (ou les) alkylglycoside(s) de formule (II) représente(nt) 35 à 50% en poids du mélange précité d'alkylglycoside.

3. Compositions selon la revendication 1 ou 2, caractérisées en ce que l'alkylglycoside de formule (II) est un oléylglycoside.

4. Compositions selon l'une des revendications 1 à 3, caractérisées en ce que dans les formules (I) et (II), G et G' sont identiques et représentent un reste de glucose et en ce que x et x' sont identiques et représentent un nombre compris entre 1,05 et 2,5.

5. Emulsions comprenant une phase aqueuse et une phase huileuse, caractérisées en ce qu'elles comprennent de 1 à 25% en poids, et de préférence de 1 à 10% en poids d'une composition émulsionnante telle que définie à l'une quelconque des revendications 1 à 4.

6. Emulsions selon la revendication 5, caractérisées en ce qu'elles comprennent, en outre, un agent émulsionnant complémentaire, en une quantité telle que la quantité totale d'agents émulsionnants au sein de l'émulsion soit inférieure ou égale à 25 % en poids.

7. Emulsions selon l'une des revendications 5 ou 6, caractérisées en ce qu'elles comprennent jusqu'à 50 % en poids d'au moins une huile choisie parmi les huiles d'origine végétale, les huiles d'origine animale, les huiles minérales et les huiles synthétiques.

8. Emulsions selon la revendication 7, caractérisées en ce que l'huile précitée est choisie dans le groupe constitué par l'huile de paraffine, l'isononanoate d'isononyle et le triheptanoate de glycérol.

9. Utilisation des émulsions selon l'une des revendications 5 à 8 pour la préparation de compositions cosmétiques.

## Patentansprüche

1. Zusammensetzungen, die insbesondere zur Herstellung fluider Emulsionen nützlich sind, umfassend:
a) 30 bis 90 Gew.-% eines Gemischs aus mindestens einem Alkylglykosid mit der Formel (I) und mindestens einem Alkylglykosid mit der Formel (II) :
RO(G)ₓ (I),
R'O(G')ₓ, (II),
worin
- R einen gesättigten oder ungesättigten, geradkettigen oder verzweigten aliphatischen Rest mit 8 bis 22 Kohlenstoffatomen bedeutet,
- R' einen Oleyl- oder Isostearylrest bedeutet,
- G und G', die gegebenenfalls verschieden sind, einen Saccharidrest bedeuten und
- x und x', die gegebenenfalls verschieden sind, eine Zahl von 1 bis 10 bedeuten,
wobei festzustellen ist, dass das/die Alkylglykosid/e mit der Formel (II) 25 bis 60 Gew.-% des Gemischs ausmacht/ ausmachen, und
b) 10 bis 70 Gew.-% mindestens eines Fettalkohols mit der Formel R₁OH, in welcher R₁ einen gesättigten oder ungesättigten, geradkettigen oder verzweigten aliphatischen Rest mit 8 bis 22 Kohlenstoffatomen bedeutet.

2. Zusammensetzungen nach Anspruch 1, **dadurch gekennzeichnet, dass** das/die Alkylglykosid/e mit der Formel (II) 35 bis 50 Gew.-% des Alkylglykosidgemischs ausmacht/ausmachen.

3. Zusammensetzungen nach Anspruch 1 oder 2, **dadurch gekennzeichnet, dass** das Alkylglykosid mit der Formel (II) ein Oleylglykosid ist.

4. Zusammensetzungen nach einem der Ansprüche 1 bis 3, **dadurch gekennzeichnet, dass** in den Formeln (I) und (II) G und G' gleich sind und einen Glucoserest bedeuten, **und dass** x und x' gleich sind und eine Zahl von 1,05 bis 2,5 bedeuten.

5. Emulsionen, die eine wässrige und eine ölige Phase umfassen, **dadurch gekennzeichnet, dass** sie 1 bis 25 Gew.-% und vorzugsweise 1 bis 10 Gew.-% einer emulgierenden Zusammensetzung wie in einem der Ansprüche 1 bis 4 definiert enthalten.

6. Emulsionen nach Anspruch 5, **dadurch gekennzeichnet, dass** sie außerdem ein komplementäres Emulgiermittel in einer derartigen Menge umfassen, dass die Gesamtmenge der Emulgiermittel in der Emulsion weniger als oder gleich 25 Gew.-% beträgt.

7. Emulsionen nach Anspruch 5 oder 6, **dadurch gekennzeichnet, dass** sie bis zu 50 Gew.-% mindestens eines Öls enthalten, das aus Ölen pflanzlichen Ursprungs, Ölen tierischen Ursprungs, Mineralölen und synthetischen Ölen ausgewählt ist.

8. Emulsionen nach Anspruch 7, **dadurch gekennzeichnet, dass** das Öl aus der Gruppe ausgewählt ist, die aus Paraffinöl, Isononylisononanoat und Glycerintrihepatanoat besteht.

9. Verwendung von Emulsionen nach einem der Ansprüche 5 bis 8 zur Herstellung kosmetischer Zusammensetzungen.

## Claims

1. Compositions, notably useful for preparing fluid emulsions, comprising :
i) 30 to 90% by weight of a mixture of at least one alkylglycoside of formula (I) and of at least one alkylglycoside of formula (II) :
RO(G)ₓ (I)
R'O(G')_{x'} (II)
in which :
- R represents a saturated or unsaturated, linear or branched aliphatic radical having 8 to 22 carbon atoms;
- R' represents an oleyl or isostearyl radical ;
- G and G', which are identical or different, represent a saccharide residue; and
- x and x', which are identical or different, represent a number between 1 and 10 ;
it being specified that the alkylglycoside(s) of formula (II) represent(s) 25 to 60% by weight of said mixture ; and
ii) 10 to 70% by weight of at least one fatty alcohol of formula R₁OH in which R₁ represents a saturated or unsaturated, linear or branched aliphatic radical having 8 to 22 carbon atoms.

2. The compositions according to claim 1, characterised in that the alkylglycoside(s) of formula (II) represent(s) 35 to 50% by weight of the above-mentioned alkylglycoside mixture.

3. The compositions according to claim 1 or 2, characterised in that the alkylglycoside of formula (II) is an oleylglycoside.

4. The compositions according to one of claims 1 to 3, characterised in that in the formulae (I) and (II), G and G' are identical and represent a glucose residue, and in that x and x' are identical and represent a number between 1.05 and 2.5.

5. Emulsions comprising an aqueous phase and an oily phase, characterised in that they comprise 1 to 25% by weight, and preferably 1 to 10% by weight, of an emulsifying composition as defined in any one of claims 1 to 4.

6. The emulsions according to claim 5, characterised in that they further comprise an additional emulsifying agent in an amount such that the total amount of emulsifying agents within the emulsion be less than or equal to 25 % by weight.

7. The emulsions according to one of claims 5 or 6, characterised in that they comprise up to 50 % by weight of at least one oil selected from oils of plant origin, oils of animal origin, mineral oils, and synthetic oils.

8. The emulsions according to claim 7, characterised in that the above-mentioned oil is selected from the group consisting of liquid paraffin, isononyl isononanoate, and glycerol triheptanoate.

9. Use of the emulsions according to one of claims 5 to 8 for preparing cosmetic compositions.
